# EUROPEAN PATENT SPECIFICATION

(11) **EP 2 267 056 B1**
(45) Date of publication and mention of the grant of the patent: **28.03.2012**
(21) Application number: 10006324.7
(22) Date of filing: 18.06.2010
(51) Int. Cl.: C08G 65/00

(54) **Process for preparing short chain polyether polyols from from ultra-low water-content starters via DMC catalysis**
Verfahren zur Herstellung von kurzkettigen Polyetherpolyolen aus Startern mit ultraniedrigem Wassergehalt mittels DMC-Katalyse
Procédé pour préparer des polyols à chaîne courte préparés à partir d'amorces à ultra faible teneur en eau via un catalyseur DMC

(30) Priority: 23.06.2009 US 489522
(43) Date of publication of application: 29.12.2010
(73) Proprietor: Bayer MaterialScience LLC, Pittsburgh, PA 15205 (US)
(72) Inventor: Pazos, Jose F., Charleston, WV25304 (US); Browne, Edward P., South Charleston, WV 25309 (US); Loveday, Anthony, South Charleston, WV 25309 (US)
(74) Representative: Klimiuk, Meike

(56) References cited:
- EP-A1- 1 577 334
- EP-A2- 1 911 787
- WO-A1-01/53381
- WO-A1-99/14258

## Description

### FIELD OF THE INVENTION

The present invention relates in general to polyether polyol production, and more specifically, to the production of short chain polyether polyols from acidified, ultra-low water content starter molecules using double metal cyanide (DMC) catalysis. The invention describes the short chain polyether polyols prepared by alkoxylating an acidified, ultra-low water content starter in the presence of an activated double metal cyanide catalyst.

### BACKGROUND OF THE INVENTION

The continuous addition of starter in a process for the preparation of polyoxyalkylene polyethers with DMC catalysts is described in U.S. Patent 5,777,177. This process continuously adds at least one starter throughout the process of preparing a polyoxyalkylene polyol. An initial starter may also be used in the process but is not required. Suitable starters to be added continuously in this process include water or low molecular weight polyols which have 1 or more hydroxyl groups and a number average molecular weight of less than 300. The polyoxyalkylene polyols prepared in the '177 patent have relatively high molecular weights, and thus low hydroxyl numbers.

U.S. Patent 5,689,012 discloses a continuous process for the preparation of polyoxyalkylene polyethers using DMC catalysts as the polyoxyalkylation catalyst employing continuous addition of alkylene oxide in conjunction with continuous addition of starter and catalyst to a continuous oxyalkylation reactor. The activated catalyst/starter mixture of Pazos et al., is stirred and heated to 105°C, and is stripped under vacuum to remove traces of water from the triol starter. All of the polyoxyalkylene polyols prepared have relatively high molecular weights and low hydroxyl numbers.

U.S. Patent 6,359,101 discloses a process for making polyether polyols. This process polymerizes an epoxide in the presence of a DMC catalyst with a first starter, with the epoxide and the first starter being continuously added to the reactor during this polymerization to form a polyol intermediate, and then reacting additional epoxide with the polyol intermediate to form the polyether polyol. Suitable compounds disclosed as first starters for this process are not the typical starters in preparing polyoxyalkylene polyols. Specific reaction conditions and impurity levels are present in this process.

An activated starter mixtures is described in U.S. Patent 6,835,801. This activated starter mixture which can be used to prepare polyoxyalkylene polyols and a process for preparing an activated starter mixture which is composed of a low molecular weight starter compound. This invention eliminates the need to synthesize costly high molecular weight starter compounds by KOH catalysis in a separate, dedicated reactor.

The direct polyoxyalkylation of glycerin and other low molecular weight starters with a DMC catalyst in a CAOS (continuous addition of starter) process is described in U.S. Patent 6,077,978. This patent describes starters such as glycerin are acid sensitive and discloses that these starters should be acidified (or similarly treated) prior to being introduced into the reactor as various impurities are present in these starters which deactivate the DMC catalyst. The effect of water in the starter feed steam on catalyst deactivation is not mentioned by U.S. Patent 6,077,978.

Commonly assigned U.S. Published Patent Application No. 2005/0209438, in the name of Brown, describes the addition of acid in an amount in excess of that needed for the mere neutralization of polyol production feed stream to produce lower molecular weight DMC-catalyzed polyols. This application is, however, silent as to the hygroscopic nature of starters such as glycerin.

Commonly assigned copending U.S. Published Patent Application No. 2008/0021191 (Reese et al) is directed to a process for the production of polyoxyalkylene polyols which is tolerant to a high water content. This process is also a DMC-catalyzed continuous addition of starter (CAOS) process. Suitable low molecular weight starters for this process are non-acid sensitive starters which contain from 200 ppm to 5,000 ppm of water and are acidified with from 10 ppm to 2,000 ppm of at least one of an inorganic protic mineral acid and an organic acid. All of the polyoxyalkylene polyols produced by this process are relatively high molecular weight and low hydroxyl number polyols.

As those skilled in the art are aware, materials such as glycerin are very hygroscopic and difficult to dry. When un-stripped glycerin, which typically has 500- 1000 ppm water, is used in a DMC-catalyzed continuous addition of starter (CAOS) process, the upper limit of the hydroxyl number is approximately 240. Deactivation of the DMC catalyst occurs beyond this point. Thus, a need exists in the art for a process for the production of short chain polyether polyols (i.e., polyols which have a hydroxyl number of greater than 250 up to 500) from hygroscopic initiators, such as glycerin, without deactivation of the DMC catalyst.

### SUMMARY OF THE INVENTION

Accordingly, the present invention provides a process for the polyoxyalkylation of a starter involving establishing oxyalkylation conditions in an oxyalkylation reactor in the presence of a double metal cyanide (DMC) catalyst, continuously introducing into the reactor at least one alkylene oxide and at least one acidified, ultra-low water content starter, wherein acid comprises greater than 100 ppm and the starter contains less than 200 ppm water, based on the weight of the starter, and recovering an oxyalkylated low molecular weight polyether polyol product. The inventive process allows the production of short chain polyether polyols, which have hydroxyl numbers of greater than 250 up to 500 and preferably of from 300 to 500, from hygroscopic initiators, such as glycerin, without deactivation of the DMC catalyst.

These and other advantages and benefits of the present invention will be apparent from the Detailed Description of the Invention herein below.

### DETAILED DESCRIPTION OF THE INVENTION

The present invention will now be described for purposes of illustration and not limitation. Equivalent weights and molecular weights given herein in Daltons (Da) are number average equivalent weights and number average molecular weights respectively, unless indicated otherwise.

As used herein, the term "polyether polyol" refers to a compound that contains at least one ether group and that contains at least one hydroxyl group.

The present invention provides a process for the polyoxyalkylation of a starter involving establishing oxyalkylation conditions in an oxyalkylation reactor in the presence of a double metal cyanide (DMC) catalyst, continuously introducing into the reactor at least one alkylene oxide and at least one acidified, ultra-low water content starter, wherein the starter contains greater than 100 ppm of acid and less than 200 ppm water, based on the weight of the starter, and recovering an oxyalkylated low molecular weight polyether product. The present invention further provides a polyether polyol made by establishing oxyalkylation conditions in an oxyalkylation reactor in the presence of a double metal cyanide (DMC) catalyst, continuously introducing into the reactor at least one alkylene oxide and at least one acidified, ultra-low water content starter, wherein the starter contains greater than 100 ppm of acid and the starter also contains less than 200 ppm water, based on the weight of the starter, and recovering the polyether polyol. The polyether polyols described herein have hydroxyl numbers of from greater than 250 up to 500 mg KOH/g, and preferably from 300 up to 500 mg KOH/g.

In the present invention, it has been unexpectedly found that by using an acidified CAOS feed stream with an ultra-low water content, it is possible to make polyether polyols which have high hydroxyl numbers (i.e. greater than 250 mg KOH/g), and thus, have of low (number average) molecular weights (Da). More specifically, these polyether polyols have hydroxyl numbers of from greater than 250 up to 500 mg KOH/g, and preferably of from 300 to 500 mg KOH/g. In addition, the functionality of these polyether polyols ranges from 1 to 8, preferably from 1 to 6, and more preferably from 2 to 4. In accordance with the present invention, the polyether polyols herein may have any combination of these upper and lower ranges of functionalities.

As used herein, the term "continuous" means a mode of addition of a relevant reactant in such a manner so as to maintain an effective concentration of the reactant substantially continuously. Continuous starter addition, for example, may be truly continuous, or may be in relatively closely spaced increments. It would not detract from the present process to incrementally add a reactant in such a manner that the added material's concentration decreases to a very low level (5-10 ppm) for some time prior to the next incremental addition. The CAOS addition may be terminated prior to completion of the alkylene oxide addition as a method decreasing the product molecular weight distribution. The catalyst may be added either initially or may be added by incremental addition. Incremental addition of reactant which does not substantially affect the nature of the product is still "continuous" as that term is used herein.

In the inventive process, polyoxyalkylene polyether polyols are prepared by the oxyalkylation of the acidified, ultra-low water content starter, in the presence of an activated double metal cyanide complex catalyst. In conventional batch processes which employ DMC catalysts, the entire initiator (or starter) is added initially to the reactor, the DMC catalyst is added, and a small percentage of the alkylene oxide feed is added. A significant pressure drop indicates that the catalyst has been activated. Alternatively, a preactivated master batch of catalyst mixed with initiator may be used. The reactor temperature is maintained at between 70°C and 150°C, and the remainder of propylene oxide added at relatively low pressure, i.e. less than 10 psig. In the conventional process, oligomeric starters having an equivalent weight in the range of 200-700 Da or higher are generally used.

In the typical CAOS process, polyoxyalkylation is accomplished by addition of a smaller amount of oligomeric starter together with catalyst and initial alkylene oxide for activation as in the conventional process. However, in the continuous addition of starter (CAOS) process, low molecular weight starter is added continuously in addition to alkylene oxide throughout the polyoxyalkylation process. The continuously added starter may be added as a separate stream or, preferably, as a mixed reactor feed stream. The amount of continuously added starter may range from 0.05 wt. % up to 30 wt. %, based on the total weight of the combined feeds (i.e. the alkylene oxide and the starter). In the CAOS process, the addition of the low molecular weight starters may be discontinued prior to the alkylene oxide feed as a method of decreasing the molecular weight distribution of the product. Another suitable method to reduce polydispersity of the polyol product is a non-CAOS step in which only alkylene oxide is added.

Additional details on the continuous addition of starter are set forth in U.S. Patent 5,777,177, the disclosure of which is hereby incorporated by reference.

Suitable starter compounds to be continuously added in accordance with the present invention include low molecular weight starters which contain one or more OH group. More specifically, these starter compounds typically have functionalities of from 1 to 8. Preferably the functionality of the starter compounds ranges from 1 to 6, and more preferably from 2 to 4. In accordance with the present invention, any combination of these upper and lower ranges of functionalities are suitable for the starter compounds herein. Examples of suitable starter compounds include methanol, ethanol, propanol, butanol, glycerin, diglycerol, ethylene glycol, propylene glycol, dipropylene glycol, trimethylolpropane, pentaerythritol, sorbitol, sucrose, etc.

As used herein, the term "ultra-low water content" with regard to the starter means a starter having a water content of less than 200 ppm, more preferably less than 150 ppm and most preferably 120 ppm or less.

Although virtually any organic or inorganic acid is suitable for use in the process of the present invention, useful acids include, but are not limited to, the mineral acids and the organic carboxylic acids, phosphonic acids, sulfonic acids, and other acids. Phosphoric acid is preferred as a mineral acid, whereas citric acid and 1,3,5-benzene tricarboxylic acids may be useful as organic acids. Acid derivatives which are reactive with bases, such as acid chlorides and acid anhydrides and the like, are also useful. Organic acids such as phosphonic acids, sulfonic acids, e.g. p-toluenesulfonic acid, and the like, may also be used. Examples of mineral acids which are suitable include hydrochloric acid, hydrobromic acid, and sulfuric acid, among others, while useful carboxylic acids or their acidifying derivatives include formic acid, oxalic acid, citric acid, acetic acid, maleic acid, maleic anhydride, succinic acid, succinic anhydride, adipic acid, adipoyl chloride, adipic anhydride, and the like. Inorganic acid precursors such as thionyl chloride, phosphorous trichloride, carbonyl chloride, sulfur trioxide, thionyl chloride phosphorus pentoxide, phosphorous oxytrichloride, and the like are considered as mineral acids herein.

As disclosed in commonly-assigned U.S. Published Patent Application No. 2005/0209438, the amount of acid added to the starter is in excess of that needed for the mere neutralization of the glycerin, i.e., greater than 100 ppm, more preferably the amount of acid ranges from greater than 100 ppm to 2,000 ppm, and most preferably 200 ppm to 300 ppm. The acid may be added to the starter used in the process of the present invention in an amount ranging between any combination of the above-recited values, inclusive of the recited values.

In theory, the upper limit on the amount of acid added to the starter is only limited by the point at which the results or properties of the resultant polyether polyol start to deteriorate.

In the continuous version of the CAOS process, the reaction may be initiated by use of an oligomeric starter, but once begun is continuously initiated by further starter. These further added starter compounds are the low molecular weight starter compounds previously disclosed herein. Alkylene oxide together with acidified, ultra-low water content starter is added at various points along the reactor which may, for example, be it a tubular reactor ("multi-point addition"), a continuous stirred tank reactor (CSTR) or a back-mixed reactor may also be used. Examples of suitable tubular reactors for the present invention include those disclosed in U.S. Published Patent 9Application No. 2004/0260056, the disclosure of which is herein incorporated by reference.

The alkylene oxides useful in the inventive process include, but are not limited to, ethylene oxide, propylene oxide, oxetane, 1,2- and 2,3-butylene oxide, isobutylene oxide, epichlorohydrin, cyclohexene oxide, styrene oxide, and the higher alkylene oxides such as the C₅-C₃₀ α-alkylene oxides. Mixtures of these alkylene oxides may also be used. Propylene oxide alone or mixtures of propylene oxide with ethylene oxide or another alkylene oxide are preferred. Other polymerizable monomers may be used as well, e.g. anhydrides and other monomers as disclosed in U.S. Patents 3,404,109, 3,538,043 and 5,145,883, the disclosures of which are hereby incorporated by references.

The process of the present invention may employ any double metal cyanide (DMC) catalyst. Double metal cyanide complex catalysts are non-stoichiometric complexes of a low molecular weight organic complexing agent and optionally other complexing agents with a double metal cyanide salt, e.g. zinc hexacyanocobaltate. Suitable DMC catalysts are known to those skilled in the art. Exemplary DMC catalysts include those suitable for preparation of low unsaturation polyoxyalkylene polyether polyols, such as disclosed in U.S. Pat. Nos. 3,427,256; 3,427,334; 3,427,335; 3,829,505; 4,472,560; 4,477,589; and 5,158,922, the disclosures of which are hereby incorporated in entirety by reference thereto. The DMC catalysts more preferred in the process of the present invention are those capable of preparing "ultra-low" unsaturation polyether polyols. Such catalysts are described in U.S. Patents 5,470,813 and 5,482,908, 5,545,601, 5,712,216, 6,689,710 and 6,764,978, the disclosures of which are hereby incorporated by references. Particularly preferred in the inventive process are those zinc hexacyanocobaltate catalysts prepared by the methods described in U.S. Patents 5,482,908 and 5,712,216, the disclosures of which are hereby incorporated in entirety by reference thereto.

The DMC catalyst concentration is chosen so as to ensure good control of the polyoxyalkylation reaction under the given reaction conditions. The catalyst concentration is preferably in the range from 0.0005 wt. % to 1 wt. %, more preferably in the range from 0.001 wt. % to 0.1 wt. %, most preferably in the range from 0.001 to 0.01 wt. %, based on the amount of polyether polyol to be produced. The DMC catalyst may be present in the process of the present invention in an amount ranging between any combination of these values, inclusive of the recited values.

The short chain polyether polyols made by the inventive process have a hydroxyl number of greater than 250 up to 500. Preferably these polyether polyols have a hydroxyl number of at least 300, and more preferably of at least 350. The hydroxyl value of the short chain polyether polyols produced by the present invention may range between any combination of these values, inclusive of the recited values.

### EXAMPLES

The present invention is further illustrated, but is not to be limited, by the following examples. All quantities given in "parts" and "percents" are understood to be by weight, unless otherwise indicated. The catalyst used in the Examples was a double metal cyanide ("DMC") catalyst made according to U.S. Patent 5,482,908.

### Example 1

In this example, an attempt was made to produce an all-propylene oxide triol of hydroxyl number 350 mg KOH/g using unstripped glycerin (i.e. having a water content of 700 ppm).

More specifically, the unstripped glycerin used in Example 1 had a water content of 700 ppm and was acidified with 240 ppm phosphoric acid.

A charge of LHT-240 (2300 g.) was charged into the reactor. LHT-240 is a triol having a hydroxyl number of 240, and a number average molecular weight of 700 Da. Next, a zinc hexacyanocobaltate catalyst (150 ppm) was charged to the reactor containing the LHT-240. The catalyst was activated by feeding a small amount of propylene oxide to the reactor, followed by heating to 130°C. A drop in pressure after several minutes indicated that the catalyst was activated. Once the catalyst was activated, two different streams (one stream of propylene oxide and a second stream of unstripped glycerin) were each fed continuously to the reactor. The total amount of propylene oxide was 12,475 g., which was fed continuously to the reactor over 5 hours. The total amount of unstripped glycerin was 3225 g., which was fed continuously to the reactor over 4 hours. Thus, the feed stream of glycerin was stopped 1 hour before the feed stream of propylene oxide was stopped.

The reactor pressure reached 40 psia near the end of the batch as the catalyst lost activity which resulted in a lot of unreacted propylene oxide. It took over an hour for the residual propylene oxide present in the reactor to cook out after the PO (propylene oxide) feed was stopped. This is considered unacceptable for a commercial process due to the catalyst being virtually deactivated.

This example illustrates the negative effect of a continuously added starter which contains more than 500 ppm of water on a DMC catalyst in a CAOS process.

### Example 2

The process and conditions of Example 1 as set forth above were repeated in Example 2, with the exception that the glycerin was nitrogen stripped to reduce the water content from 700 ppm to less than 200 ppm. As in Example 1, the catalyst concentration was 150 ppm and the feed time was 5 hours for propylene oxide and 4 hours for glycerin. Thus, as in Example 1, the feed stream of glycerin was stopped 1 hour before the feed stream of propylene oxide was stopped.

The glycerin in Example 2 contained less than 200 ppm of water and was acidified with 240 ppm phosphoric acid.

In Example 2, the reactor pressure never reached 20 psia, and the catalyst maintained its activity throughout the batch. The cookout of unreacted propylene oxide after the feed stream was discontinued was very fast, which indicated only a small amount of unreacted propylene oxide was present and that the DMC catalyst was very active throughout the process.

The polyoxyalkylene polyol produced in Example 2 was an all-propylene oxide triol having a hydroxyl number of 350 mg KOH/g.

The foregoing examples of the present invention are for the purpose of illustration and not limitation. It will be apparent to those skilled in the art that the embodiments described herein may be modified or revised in various ways without departing from the spirit and scope of the invention. The scope of the invention is to be measured by the appended claims.

## Claims

1. A process for the polyoxyalkylation of a starter comprising:
(a) establishing oxyalkylation conditions in an oxyalkylation reactor in the presence of a double metal cyanide (DMC) catalyst;
(b) continuously introducing into the reactor at least one alkylene oxide and at least one acidified, ultra-low water content starter, wherein the starter contains greater than 100 ppm acid and less than 200 ppm water, based on the weight of the starter;
and
(c) recovering an oxyalkylated low molecular weight polyether product wherein said polyether polyol has a hydroxyl number of from greater than 250 up to 500 mg KOH/g.

2. The process according to Claim 1, wherein the starter is selected from the group consisting of methanol, ethanol, propanol, glycerin, diglycerol, polyglycerol, ethylene glycol, propylene glycol, dipropylene glycol, trimethylolpropane, pentaerythritol, sorbitol and sucrose.

3. The process according to Claim 1, wherein the starter is acidified with an acid selected from the group consisting of mineral acids, organic carboxylic acids, phosphonic acids, sulfonic acids and combinations thereof.

4. The process according to Claim 1, wherein the acid is selected from the group consisting of citric acid, 1,3,5-benzene tricarboxylic acids, phosphonic acids, p-toluenesulfonic acid, hydrochloric acid, hydrobromic acid, sulfuric acid, formic acid, phosphoric acid, oxalic acid, citric acid, acetic acid, maleic acid, maleic anhydride, succinic acid, succinic anhydride, adipic acid, adipoyl chloride, adipic anhydride, thionyl chloride, phosphorous trichloride, carbonyl chloride, sulfur trioxide, thionyl chloride phosphorus pentoxide, phosphorous oxytrichloride and combinations thereof.

5. The process according to Claim 1, wherein the alkylene oxide is selected from the group consisting of ethylene oxide, propylene oxide, 1,2- and 2,3-butylene oxide, isobutylene oxide, epichlorohydrin, cyclohexene oxide, styrene oxide and C₅-C₃₀ α-alkylene oxides.

6. The process according to Claim 1, additionally comprising stopping the introduction of said at least one acidified, ultra-low water content starter into the reactor in step (b) and continuing to introduce at least one alkylene oxide into the reactor.

## Patentansprüche

1. Verfahren zur Polyoxyalkylierung eines Starters, bei dem man:
(a) in einem Oxyalkylierungsreaktor in Gegenwart eines Doppelmetallcyanid-Katalysators (DMC-Katalysators) Oxyalkylierungsbedingungen einstellt;
(b) in den Reaktor kontinuierlich mindestens ein Alkylenoxid und mindestens einen angesäuerten Starter mit ultraniedrigem Wassergehalt einträgt, wobei der Starter mehr als 100 ppm Säure und weniger als 200 ppm Wasser, bezogen auf das Gewicht des Starters, enthält;
und
(c) ein oxyalkyliertes niedermolekulares Polyetherprodukt gewinnt, wobei das Polyetherpolyol eine Hydroxylzahl von mehr als 250 bis 500 mg KOH/g aufweist.

2. Verfahren nach Anspruch 1, bei dem man den Starter aus der Gruppe bestehend aus Methanol, Ethanol, Propanol, Glycerin, Diglycerin, Polyglycerin, Ethylenglykol, Propylenglykol, Dipropylenglykol, Trimethylolpropan, Pentaerythrit, Sorbit und Saccharose auswählt.

3. Verfahren nach Anspruch 1, bei dem man den Starter mit einer Säure aus der Gruppe bestehend aus Mineralsäuren, organischen Carbonsäuren, Phosphonsäuren, Sulfonsäuren und Kombinationen davon ansäuert.

4. Verfahren nach Anspruch 1, bei dem man die Säure unter Citronensäure, 1,3,5-Benzoltricarbonsäuren, Phosphonsäuren, p-Toluolsulfonsäure, Chlorwasserstoffsäure, Bromwasserstoffsäure, Schwefelsäure, Ameisensäure, Phosphorsäure, Oxalsäure, Citronensäure, Essigsäure, Maleinsäure, Maleinsäureanhydrid, Bernsteinsäure, Bernsteinsäureanhydrid, Adipinsäure, Adipolychlorid, Adipinsäureanhydrid, Thionylchlorid, Phosphortrichlorid, Carbonylchlorid, Schwefeltrioxid, Thionylchlorid, Phosphorpentoxid, Phosphoroxidtrichlorid und Kombinationen davon auswählt.

5. Verfahren nach Anspruch 1, bei dem man das Alkylenoxid unter Ethylenoxid, Propylenoxid, 1,2-und 2,3-Butylenoxid, Isobutylenoxid, Epichlorhydrin, Cyclohexenoxid, Styroloxid und C₅-C₃₀-α-Alkylenoxiden auswählt.

6. Verfahren nach Anspruch 1, bei dem man zusätzlich das Eintragen von mindestens einem angesäuerten Starter mit ultraniedrigem Wassergehalt in den Reaktor in Schritt b) stoppt und mit dem Eintragen von mindestens einem Alkylenoxid in den Reaktor fortfährt.

## Revendications

1. Procédé pour la polyoxyalkylation d'un composé de départ comprenant :
(a) l'établissement de conditions d'oxyalkylation dans un réacteur d'oxyalkylation en présence d'un catalyseur à cyanure de métal double (DMC) ;
(b) l'introduction en continu dans le réacteur d'au moins un oxyde d'alkylène et d'au moins un composé de départ à très faible teneur en eau acidifié, où le composé de départ contient plus de 100 ppm d'acide et moins de 200 ppm d'eau, sur la base du poids du composé de départ ;
et
(c) la récupération d'un produit polyéther à faible poids moléculaire oxyalkylé où ledit polyéther polyol a un indice d'hydroxyle de plus de 250 jusqu'à 500 mg KOH/g.

2. Procédé selon la revendication 1, dans lequel le composé de départ est choisi dans le groupe constitué des méthanol, éthanol, propanol, glycérine, diglycérol, polyglycérol, éthylèneglycol, propylèneglycol, dipropylèneglycol, triméthylolpropane, pentaérythritol, sorbitol et saccharose.

3. Procédé selon la revendication 1, dans lequel le composé de départ est acidifié avec un acide choisi dans le groupe constitué d'acides minéraux, d'acides carboxyliques organiques, d'acides phosphoniques, d'acides sulfoniques et de combinaisons de ceux-ci.

4. Procédé selon la revendication 1, dans lequel l'acide est choisi dans le groupe constitué de l'acide citrique, les acides 1,3,5-benzènetricarboxyliques, les acides phosphoniques, l'acide p-toluènesulfonique, l'acide chlorhydrique, l'acide bromhydrique, l'acide sulfurique, l'acide formique, l'acide phosphorique, l'acide oxalique, l'acide citrique, l'acide acétique, l'acide maléique, l'anhydride maléique, l'acide succinique, l'anhydride succinique, l'acide adipique, le chlorure d'adipoyle, l'anhydride adipique, le chlorure de thionyle, le trichlorure phosphoreux, le chlorure de carbonyle, le trioxyde de soufre, le chlorure de thionyle, le pentoxyde de phosphore, l'oxytrichlorure de phosphore et des combinaisons de ceux-ci.

5. Procédé selon la revendication 1, dans lequel l'oxyde d'alkylène est choisi dans le groupe constitué de l'oxyde d'éthylène, l'oxyde de propylène, l'oxyde de 1,2- et 2,3-butylène, l'oxyde d'isobutylène, l'épichlorhydrine, l'oxyde de cyclohexène, l'oxyde de styrène et des oxydes de α-alkylène en C₅-C₃₀.

6. Procédé selon la revendication 1, comprenant en outre l'arrêt de l'introduction dudit au moins un composé de départ à très faible teneur en eau acidifié dans le réacteur dans l'étape (b) et la continuation de l'introduction d'au moins un oxyde d'alkylène dans le réacteur.
